# EUROPEAN PATENT APPLICATION

(11) **EP 4 574 177 A1**
(43) Date of publication of application: **25.06.2025**
(21) Application number: 24220084.8
(22) Date of filing: 16.12.2024
(51) Int. Cl.: A61L 2/07, A61B 50/33, A61B 90/70, A61C 19/00, A61B 50/00

(54) **CASSETTE FOR STERILISATION OF INSTRUMENTS FOR MEDICAL USE AND METHOD FOR STERILISATION OF SAID INSTRUMENTS FOR MEDICAL USE**

(30) Priority: 19.12.2023 IT 202300027285
(71) Applicant: Bonanni, Marco, 35142 Padova (PD) (IT)
(72) Inventor: Bonanni, Marco, 35142 Padova (PD) (IT)
(74) Representative: Gallo, Luca

(57) **Abstract**

A cassette for the sterilisation of instruments for medical use, comprising two containment bodies (2, 3) coupled together to delimit a housing chamber (100) containing a support tray (4) for supporting the instruments to be sterilised. One of the two bodies being provided with at least one opening (32A) closed by a vapour-permeable sheet-like element (5) shaped to interpose itself between a first frame (32) delimiting the opening and a second frame (40) of the tray (4) to seal the container flow passage (V) of steam during an intended saturated steam autoclave sterilisation procedure.

## Description

### Field of application

The present invention relates to a cassette for sterilisation of instruments for medical use and a method for sterilisation of said instruments, according to the preamble to the respective independent claims 1 and 7.

The cassette and the method for sterilisation in question are advantageously intended for use in the sterilisation of instruments for medical use of various kinds, in particular in the fields of surgery and dentistry. Instruments for medical use, such as, for example, mirrors, scalpels, burs, suction cannulas, specils, etc., which are intended to come into contact with the human body or with potentially infectious fluids and organs of the human body, must undergo complete sterilisation in order to be reused.

Therefore, the present invention fits into the industrial sector of the production of instruments for the sterilisation of instruments for medical use, which can be used in professional medical practices, such as dental practices, or even in hospitals.

### State of the art

Surgical cassettes are known on the market and are regularly used in the surgical and orthodontic sector to contain instruments and to subject them to sterilisation treatments or even preventive disinfection.

More in detail, known surgical cassettes are usually made up of a container, generally metal, delimiting a containment compartment inside which can be used to house surgical or orthodontic instruments, hereafter and more generally referred to as `instruments for medical use', such as mirrors, scalpels, burs, suction cannulae, mirrors, etc. This well-known surgical cassette is equipped with wide perforated walls to allow the passage of treatment fluids and more specifically for the passage of steam during a planned sterilisation phase or also for the passage of cleaning and disinfection liquids during a possibly preventive washing phase in a special equipment for cleaning with high temperature or ultrasound liquids.

Operationally, the known surgical cassette described above is usually employed as part of a sterilisation method for medical instruments as detailed below.

The medical instruments are first individually identified and then undergo a cleaning phase to remove residual dirt from the instruments that could render the subsequent sterilisation phase ineffective. The cleaning phase can be performed by manual or mechanical washing. Mechanical washing can advantageously involve placing the instruments for medical use in the aforementioned surgical cassette in a known manner and then placing the latter in ultrasound or thermodisinfection equipment that allows for the physical and chemical disintegration of the bacterial contaminants contained in the dirt residues and thus their removal.

Once the cleaning phase is complete, the cassette is placed inside a sterilising bag for the sterilisation phase, which aims to eliminate all microbial forms (pathogenic and non-pathogenic, including spores and fungi) from the medical instruments used. This sterilisation phase is implemented through the use of sterilising equipment, in particular steam autoclaves, which are devices that guarantee the sterilisation of medical instruments generally through a combination of pressure, saturated steam temperature and exposure time.

The sterilisation pouch consists of a transparent sheet of airtight plastic material, sealed on three edges to a sheet of medical paper, which will act as a filter for the passage of the sterilised element (e.g. steam). After inserting the cassette inside this envelope, the fourth edge is sealed and the envelope is placed inside the sterilising equipment.

As an alternative to using sterilising pouches, the operator can start with a tubular sterilisation roll and, using a sealing machine, cut and seal two end edges, thus creating closed pouches.

As a further alternative to rolls or envelopes for sterilisation, a suitably sized sheet of medical paper can be used, appropriately wrapped around the cassette and sealed with tape so that there is no direct passage of air. During the sterilisation phase, a steam passage stage takes place, during which the pores of the paper allow steam to pass through, which acts as a sterilising fluid, while in the subsequent drying stage the pores close, making the sheet of paper airtight and thus guaranteeing that sterilisation is maintained over time.

On leaving the sterilisation equipment, the operator in charge visually checks the integrity of the pouch before storing it.

The known type surgical cassette described above, once removed from the sterilisation equipment, is then used for transport from the sterilisation room to the place of use; for this reason, the surgical cassette is equipped with a safety lock to prevent accidental opening.

The surgical cassette and the sterilisation method thus conceived in accordance with the technique known to date are not without drawbacks.

Firstly, the above method requires a lot of staff time in the sterilisation room and also requires that all steps are carried out with extreme care and attention to prevent the contents of the surgical cassette from being contaminated after sterilisation.

Secondly, it should be noted that the surgical cassette used in known sterilisation methods is brought into the work area and opened just before the medical instruments contained in it are used. At this point, all the wrapping material, consisting of medical paper or medical paper and plastic film, is removed from the surgical cassette and sorted into the appropriate containers for disposal. The amount of wrapper material removed depends on the size of the surgical cassette and is generally quite bulky. As the sterilisation pouches are made of two different materials, they must be handled by correctly separating the paper from the plastic in order to be recycled, thus wasting additional resources in terms of time and personnel.

A further drawback of known types of surgical cassettes is that insofar as they are made of metal material, their contents are not visible and, therefore, a label must be placed on the outside of the envelope or a coding must be provided (e.g. a colour coding of the outside of the envelope) so that the type of instrument housed inside the surgical cassette can be identified from the outside. The label must be visible from the outside of the pouch and must not break or peel off, so that useful information is not lost when that particular instrument is needed. These requirements are a complication of the sterilisation method in use today in accordance with the known art.

An alternative to cassettes, which is very much in use in dental practice, is to realise the method of sterilisation of dental instruments by packing them directly into pouches or using sterilisation rolls. In some cases, some clinics eliminate the outer wrapping by sterilising loose medical instruments directly by placing them inside the sterilisation equipment without the use of any surgical cassettes or by placing the instruments directly on trays.

The latter method of sterilisation defines a procedure that is not recommended and is only acceptable if the instruments for medical use once sterilised are used immediately, without being stored for a period of non-use.

The use of rolls or pouches to make the outer wrapping of single medical instruments or those collected in small groups is a practice that allows one to see inside the wrappings and to use only the material that forms the wrappings for the quantity that is needed. The drawback of this procedure in the relevant sterilisation method is that it considerably increases the time required to prepare the wrapping and that a significant quantity of rolls or envelopes is used to make the wrapping, with the consequent need to sort out as many of these materials for recycling.

Furthermore, the storage of medical instruments directly in envelopes or rolls and not in surgical cases exposes the instruments themselves to a risk of damage during transport on the one hand and a risk of injuring the transport operator on the other hand, with the additional inconvenience

### Presentation of the invention

In this situation, the problem underlying the present invention is to obviate the drawbacks manifested by the known technique described above by providing a cassette for sterilising instruments for medical use and a method for sterilising such instruments which are practical and make it possible to simplify and speed up the sterilisation step itself.

A further scope of the present invention is to provide a cassette for sterilisation of instruments for medical use and a method for sterilisation of such instruments which are economical.

Another object of the present invention is to provide a cassette for sterilisation of instruments for medical use and a method for sterilising such instruments which is completely safe for the sterilisation personnel or for the personnel in charge of using the instruments by taking them from the surgical cassettes.

Another purpose of the present invention is to provide a cassette for sterilising instruments that is easy to assemble for the subsequent sterilisation phase.

Another purpose of the present invention is to provide an instrument sterilisation cassette which allows for easy disposal of consumable parts used in each sterilisation cycle.

Another purpose of the present invention is to provide an instrument sterilisation cassette which is easy to assemble for the subsequent sterilisation step.

### Brief description of the drawings

The technical characteristics of the invention, according to the aforementioned purposes, are clearly apparent from the content of the claims below, and the advantages thereof will be more evident in the detailed description below, made with reference to the accompanying drawings, which represent two purely illustrative and non-limiting embodiments thereof, in which:
- figure 1 shows a perspective view of a cassette that is the subject of the present invention in accordance with a first implementation form;
- figure 2 shows the cassette of figure 1 with some parts removed (the second containment body functioning as a lid) to better highlight others;

- figure 3 shows a detail of the cassette of figure 1 relating to a first containment body;
- figure 4 shows a detail of the cassette in figure 1 relating to a second containment body;
- the figure 5 shows a detail of the cassette of figure 1 relative to a tray for the support of instruments for medical use;
- figure 6 shows a perspective view of a cassette which is the subject of the present invention in accordance with a second design and with the first containment body in a partially raised position from the second containment body;
- figure 7 shows the cassette of figure 1 in exploded view;
- figure 8 shows the cassette of figure 1 in a closed configuration and in a cross-sectional view made along a transverse plane orthogonal to the containment bodies;
- figure 9 shows an enlarged detail of the cassette of figure 8;

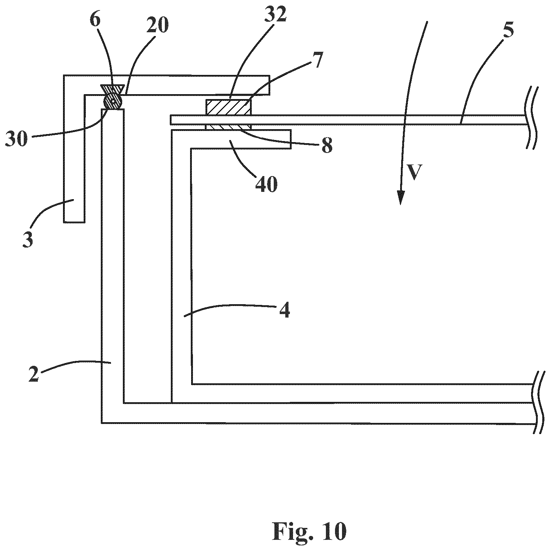

- figure 10 shows an enlarged detail of the cassette of figure 1 illustrated in a schematic section to highlight the position of the seals.

### Detailed description of a preferred embodiment

With reference to the accompanying drawings, the cassette for sterilisation of instruments for medical use which is the subject matter of the present invention has been designated as 1.

The cassette which is the subject matter of the present invention can be used in any context where it is desired to subject instruments to an autoclave sterilisation procedure, or to subject instruments to sterilisation procedures by means of steam at high temperature.

The cassette for the sterilisation of instruments for medical use will advantageously be used in an illustrative but not limitative manner for the sterilisation of instruments for medical use that can be used in the field of surgery and orthodontics. In any case, the term 'instruments for medical use' is to be understood hereafter to mean any instrument intended to come into contact with animal organs or fluids and which, for the purpose of reuse, must be subjected to sterilisation, such as burs, specils, scalpels, cannulas, handpieces, mirrors etc. for orthodontic use or such as scalpels, scissors etc. for surgical use.

The cassette 1 comprises a first containment body 2 provided with a first peripheral edge 20 and a second containment body 3 provided with a second peripheral edge 30.

The second containment body 3 is provided with at least one wall 31 on which is provided at least one first fluid passage opening 32A delimited by a corresponding first frame 32. Advantageously, said wall 31 corresponds in accordance with the illustrated embodiments to a bottom wall. In accordance with the second embodiment, said bottom wall of the second containment body 3 defines a lid which, in accordance with a not illustrated embodiment example, could have an entirely flat development.

Advantageously, the two containment bodies 2 and 3 are both provided with a rectangularly shaped bottom wall 21, 31 in accordance with both embodiments illustrated in the accompanying drawings. Advantageously, the aforementioned bodies 2 and 3 are also provided with a perimeter side wall 22, 32 extending from the respective end wall 21, 31 preferably orthogonally.

The two containment bodies 2, 3 are shaped to mate with each other defining a closed configuration A for said cassette 1, in which they enclose chamber 100 for the containment of medical instruments as further specified below.

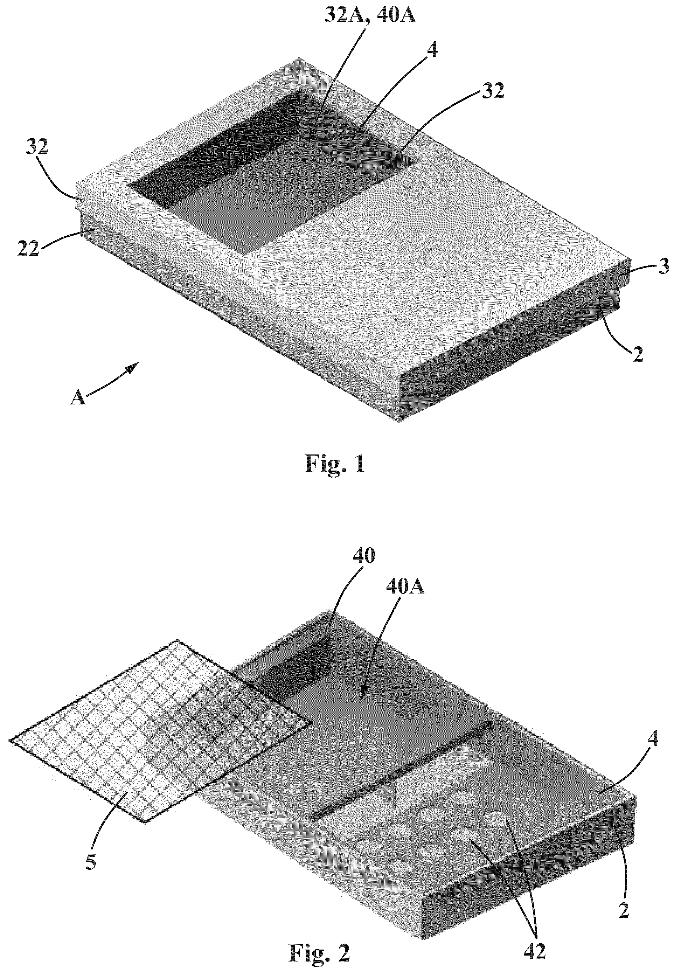

In such a closed configuration A (see figure 1) of the cassette 1 the respective first peripheral edge 20 of the first containment body 2 and second peripheral edge 30 of the second containment body 3 are opposed to each other.

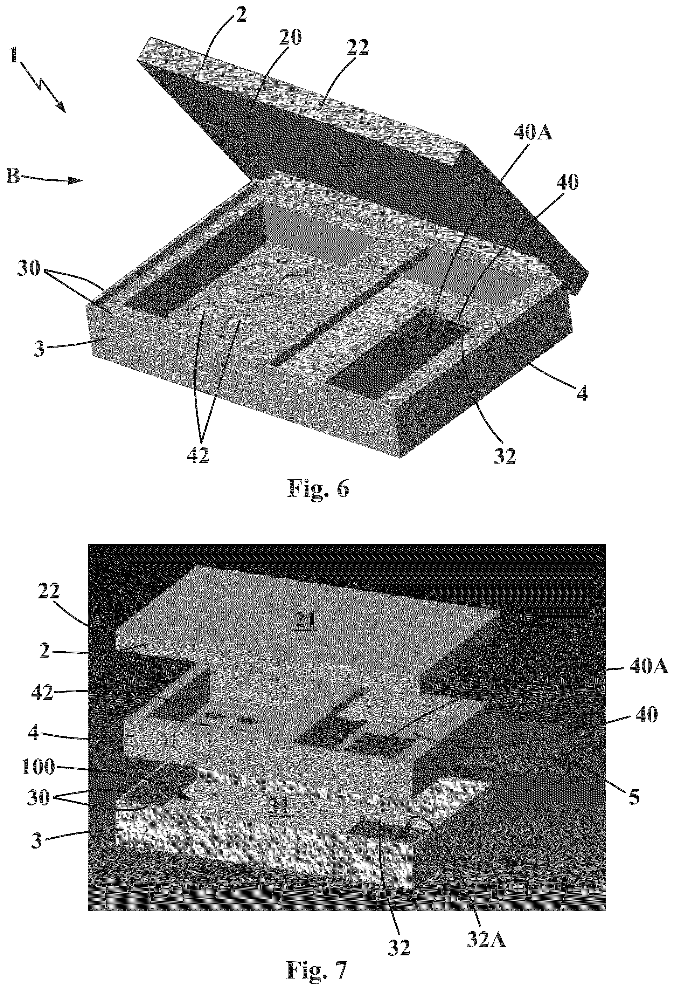

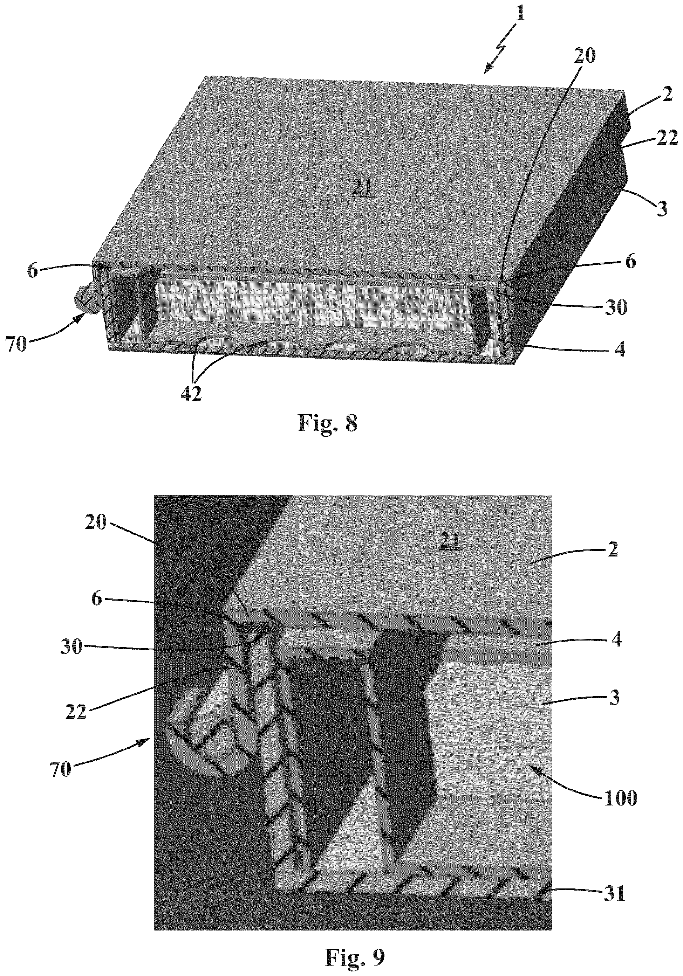

The two bodies are mutually movable so that the cassette 1 assumes the open configuration B for example illustrated in figure 6, in which the first peripheral edge 20 and the second peripheral edge 30 of the two containment bodies 2 and 3 are spaced apart from each other, wherein spaced apart means that the bodies are either completely separated (in accordance with the first embodiment) or partially separated by being pivotally engaged with each other by means of, for example, one or more hinges 70 (in accordance with the second embodiment, see figures 8 and 9).

The cassette 1 according to the present invention further comprises at least one support tray 4, suitable for supporting one or more instruments for medical use (not illustrated in the appended figures as they are in themselves well known to the person skilled in the art), provided with at least one second frame 40 delimiting a second fluid passage opening 40A.

The support tray 4 being shaped in shape and in size to be housed within the housing chamber 100. When the cassette 1 is in a closed configuration A with the tray 4 housed in the chamber, the first and second frames 32, 40 are opposed to each other with the first opening 32A and the second opening 40A in communication with each other to delimit a flow passage V for a sterilising fluid. Advantageously, in accordance with the examples of the enclosed figures, the two openings 32A and 40A are substantially equal in size and are at least partially overlapping and preferably aligned and substantially overlapping with each other to define the flow passage V, that is, the steam passage channel during the sterilisation phase inside the autoclave as will be better clarified below.

The cassette 1 according to the present invention, further comprising at least one vapour-permeable sheet-like element 5, which is shaped to be interposed when the cassette 1 is in the closed configuration A, between the first frame 32 of the second containment body 3 and the second frame 40 of the tray 4, in order to seal off the flow passage V.

The material of which the sheet-like element 5 is composed can be of a traditional type and well known to the technician of the sector, in particular usually defined functionally as a sheet of medical paper which acts as a filter for the passage of the sterilised element in particular steam. During the sterilisation phase, a steam passage stage takes place, during which the pores of the paper allow the passage of steam that acts as a sterilising fluid, while in the subsequent drying stage the pores close, making the sheet of paper hermetic and thus guaranteeing the maintenance of sterilisation over time.

The cassette 1 according to the present invention further comprises first sealing means 6 which are interposed between the first peripheral edge 20 of the first containment body 2 and the second peripheral edge 30 of the second containment body 3. Thanks to this seal, steam can enter and exit during the sterilisation phase in the autoclave exclusively from the flow passage V, while this seal together with the behaviour of the sheet-like element 5, guarantee the preservation of the sterilisation once the cassette 1 is removed from the autoclave.

In accordance with a preferred feature of the present invention there are provided second sealing means 7 interposed between the first frame 32 of the second containment body 3 and an opposing perimeter band of the sheet-like element 5.

The aforementioned first sealing means comprise, for example, a first annular seal fixed to the first peripheral edge 20 of the first containment body 2 or to the second peripheral edge 30 of the second containment body 3.

In accordance with an embodiment of the invention, the first sealing means 6 may comprise a first seat (not illustrated) obtained on the first peripheral edge 20 of the first containment body 2 or on the second peripheral edge 30 of the second containment body 3, and a first annular seal housed in said first seat.

There are also advantageously third sealing means 8 interposed between the second frame 40 of the support tray 4 and an opposing peripheral band of the foil element 5.

One or both of the aforementioned second and third sealing means 7, 8 are fixed to at least one corresponding face of the sheet-like element 5 in correspondence with its perimeter band, being for example obtained by an increased thickness of the same sheet-form element or by a printing of insulating material obtained on one face or preferably on both faces of the aforementioned sheet-like element 5 in correspondence with the aforementioned perimeter band.

Otherwise, in accordance with a further embodiment the second sealing means 7 are fixed to the first frame 32 of the second containment body 3 being for this purpose preferably made of plastic material. The moulding may be carried out, for example, by co-moulding two different plastic materials of which one is more rigid to make the containment body 3 and one is softer and elastically yielding to make the second sealing means 7 in the form of a second annular seal.

In accordance with a variant embodiment, the second sealing means 7 may comprise a second seat (not illustrated) obtained on the first frame 32 of the second containment body 3, and a second annular seal housed in said first seat.

Mutatis mutandis, similarly to what has been described above with reference to the second sealing means 7, the third sealing means 8 may be fixed to the second frame 40 of the tray 4, the latter being for this purpose preferably made of plastic material. The moulding may be carried out, for example, by co-moulding two different plastic materials of which one is more rigid to make the tray 4 and one is softer and elastically yielding to make the third sealing means 8 in the form of a third annular seal.

In accordance with a variant embodiment, the third sealing means 8 may comprise a third seat (not illustrated) formed on the second frame 40 of the tray 4, and a third annular gasket housed in that third seat.

Advantageously, all seals are made of biocompatible material.

In accordance with the examples illustrated in the attached figures, the tray 4 comprises one or more shaped portions 42 for removably retaining medical instruments. Such portions may be in the form of holes or coupling elements for direct retention of medical instruments, or in the form of grids or other shaped form for supporting frames e.g. made of silicone on which medical instruments may be housed.

In accordance with an advantageous feature of the invention, at least one between the first containment body 2 and the second containment body 3 comprises at least one transparent portion opposed to the shaped portion of the tray 4 for seeing externally to the cassette 1 the shaped portion 42 of the tray with the instruments for medical use to be sterilised on it, so as to always have easy knowledge of what has been sterilised.

Obviously without departing from the scope of protection of the present design, the tray for the support of instruments for medical use may take various forms and may be made of one or more distinct elements which may be modular with each other and in one and the same material, preferably plastic or in several materials.

In turn, the cassette 1, will be advantageously shaped, i.e. it will have a substantially standardized shape and dimensions, so that it can be easily inserted in equipment for the mechanical cleaning of medical instruments by means of ultrasound or washing with high temperature liquids.

In accordance with the embodiment illustrated in Figures 8 and 9, the first containment body 2 and the second containment body 3 are engaged with each other by means of a hinge 70, being rotatably movable with respect to each other between an open configuration B for inserting the tray 4 between the first and second containment bodies 2, 3, and a closed configuration A for delimiting the housing chamber 100 with the tray 4 inside.

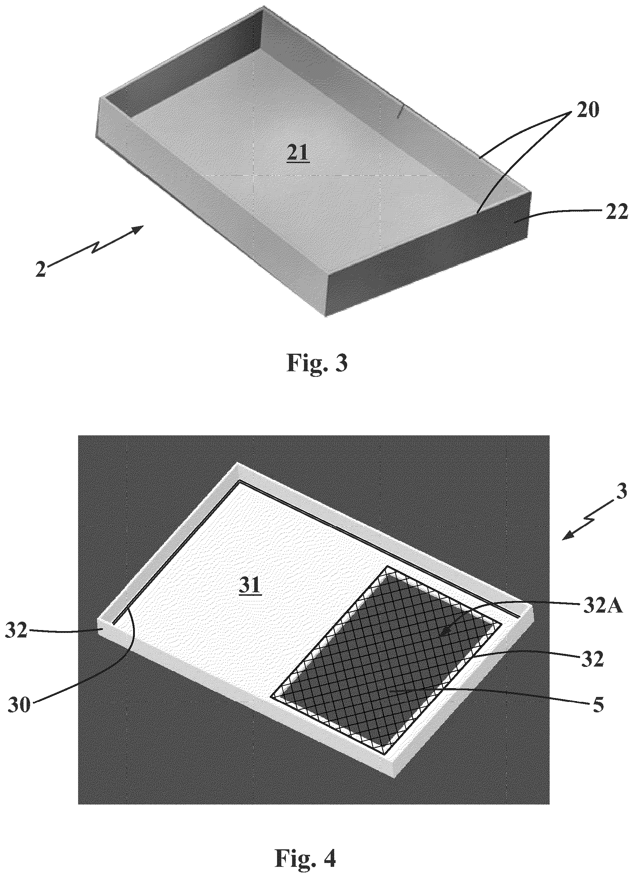

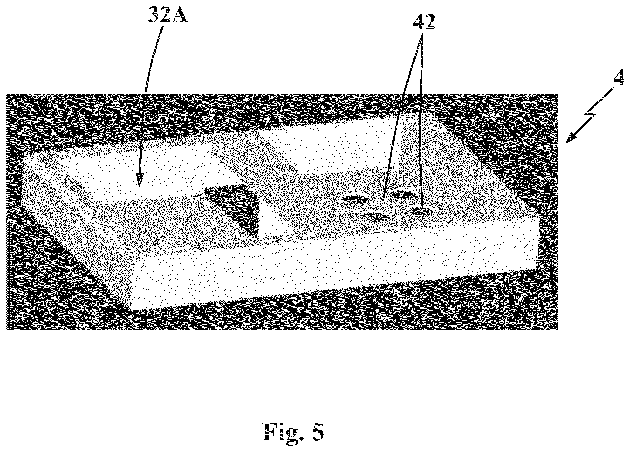

In accordance with the embodiment illustrated in Figures 1 to 5, the second containment body 3 is in the form of a lid coupled to the first containment body 2. In this example, the first containment body 2 is provided with a first perimeter wall 22 extending from a first closed bottom wall 21. The second containment body 3 is provided with a second perimeter wall 32 arranged externally to the first perimeter wall 22 of the first containment body 2 and developed from a second end wall 31 provided with the first opening 32A.

In accordance with the embodiment illustrated in Figures 6-9, the first containment body 2 is in the form of a lid coupled to a closure on the second containment body 3. In this example, the first containment body 2 is provided with a first perimeter wall 22 extending from a first closed bottom wall 21. The second containment body 3 is provided with a second perimeter wall 32 arranged internally to the first perimeter wall 22 of the first containment body 2 and developed from a second bottom wall 31 provided with the first opening 32A.

It is also an object of the present invention to provide a method for the sterilization of instruments for medical use using a cassette as described above, references to which will be retained below for simplicity of exposition.

The method object of the present invention comprises the following operational steps.

Initially, there is a step of preparing a tray 4 with one or more medical instruments to be sterilized supported on it. In this phase, the instruments are loaded into the appropriate seats (shaped portions 42) of the tray 4 either directly or by means of frames or supports, for example made of silicone.

This is followed by a phase in which the cassette 1 is modified from an initially open configuration, which allows the insertion of the tray 4 into it, to a closed position in which it delimits the housing chamber 100 (i.e. for sterilization of the instruments). This change in configuration takes place by coupling the first and second containment bodies and achieving the closed position A with the tray 4 supporting the medical instruments to be sterilized being housed within the housing chamber 100.

Said coupling of the two containment bodies 2, 3 thus determines the formation of a cassette set up with instruments for medical use to be sterilized and determines the contraposition between them of the first peripheral edge 20 and the second peripheral edge 30 respectively of the first and second containment bodies 2, 3 with the interposition of the first sealing means 6. Said coupling of the two containment bodies 2, 3 also determines the juxtaposition between them of the first and second frames 32, 40 respectively of the second containment body 32 and the tray 4 with the interposition of at least one vapor-permeable sheet-like element 5 to seal in a sealed manner the flow passage V delimited by the first opening 32A and the second opening 40A of the first and second frames 32, 40.

At this point, once the cassette has been set up, it is placed in the sterilization chamber of an autoclave sterilization apparatus and an autoclave sterilization procedure is initiated (after closing the relevant door) in which a fluid in the state of steam passes through the flow passage V and sterilizes the medical instruments supported by the tray 4, thereby obtaining a cassette set up with sterilized medical instruments.

This is followed by an extraction phase of the cassette with sterilized medical instruments from the sterilization chamber of the autoclave.

Advantageously, the method provides that the tray 4 containing the medical instruments still soiled (i.e., possibly after manual cleaning) is subjected, before being inserted into the cassette containment chamber, to mechanical washing operations by inserting the decayed medical instruments with residual soiling in a mechanical washing apparatus such as, for example, a high-temperature or ultrasonic liquid washing apparatus. Only then is the tray with the washed or even disinfected instruments inserted into the chamber 40 of the closed cassette 1, as specified above, by use of the sheet-like element 5.

Advantageously, the aforementioned method in accordance with the embodiment of the cassette illustrated in Figures 1-5 envisages that the juxtaposition of the first and second frames 32, 40 with the interposition of the vapor-permeable sheet-like element 5 takes place by first arranging the sheet-like element 5 on the second frame 40 of the tray 4 and then with the subsequent superimposition of the second containment body 3 in the form of a lid with the first frame 32 on the sheet-like element, as well as obviously with the simultaneous superimposition of the two peripheral edges 20, 30 of the two containment bodies 2, 3.

Advantageously, the aforementioned method in accordance with the embodiment form of the box illustrated in Figures 6-9 envisages that the contraposition between them of the first and second frames 32, 40 with the interposition of the vapor-permeable sheet-like element 5 takes place by first arranging the sheet-like element 5 on the second botton wall 31 of the second containment body 3 in correspondence with the first opening 32A, and then superimposing on the second botton wall 31 with the interposition of the sheet-like element 5 the tray 4 so that the first and second frames 32, 40 are opposed to each other. This is followed by closing the cassette with the arrangement of the first containment body 2 on the second containment body 3 with overlapping of the two peripheral edges 20, 30.

The invention thus conceived therefore achieves its intended purposes.

## Claims

1. Cassette for the sterilization of instruments for medical use, **characterized in that** it comprises:
- a first containment body (2) provided with at least a first peripheral edge (20);
- a second containment body (3) provided with at least a second peripheral edge (30) and at least one wall (31) provided with at least a first opening (32A) for fluid passage delimited by a first frame (32); said first containment body (2) and said second containment body (3) being shaped to mutually couple to define a closed configuration (A) of said cassette (1), in which they delimit a housing chamber (100) for instruments for medical use and in which the respective first peripheral edge (20) and second peripheral edge (30) are opposite each other;
- at least a support tray (4) suitable for supporting one or more instruments for medical use, equipped with at least a second frame (40) delimiting a second opening (40A) for fluid passage, said support tray being shaped to be housed in said housing chamber (100) with said first and second frames (32, 40) opposite each other and with said first opening (32A) and second opening (40A) in communication with each other to delimit a flow passage (V) for a fluid sterilizing with said cassette (1) in said closed configuration (A);
- at least one vapor-permeable sheet-like element (5), shaped to be interposed between the first frame (32) of said second containment body (3) and the second frame (40) of said support tray (4) to seal said container flow passage (V) with said cassette (1) in said closed configuration (A);
- first sealing means (6) being provided interposed between the first peripheral edge (20) of said first containment body (2) and the second peripheral edge (30) of said second containment body (3) with said cassette (1) in said closed configuration (A).

2. Cassette for the sterilization of instruments for medical use according to claim 1, **characterized in that** of comprising second sealing means (7) interposed between the first frame (32) of said second containment body (3) and said sheet-like element (5) and/or third sealing means (8) placed between the second frame (40) of said tray (4) and said sheet-like element (5).

3. Cassette for the sterilization of instruments for medical use according to claim 2, **characterized in that** at least one of said second or third sealing means (7, 8) are fixed to at least one corresponding face of said sheet-like element in correspondence with one its perimetral band.

4. Cassette for the sterilization of instruments for medical use according to any of the previous claims, **characterized in that** of being shaped for insertion into mechanical cleaning equipment by ultrasonic or by washing with high temperature liquids.

5. Cassette for the sterilization of instruments for medical use according to any of the previous claims, **characterized in that** said second containment body (3) is in the form of a lid which can be coupled onto said first containment body (2) to define said configuration of closure (A); said first containment body (2) being provided with a first perimetral wall (22) which extends from a first closed bottom wall (21); said second containment body (3) being provided with a second perimetral wall (32) arranged externally to the first perimeter wall (22) of said first containment body (2) and which extends from a second bottom wall (31) provided with said first opening (32A).

6. Cassette for the sterilization of instruments for medical use according to any of the previous claims from 1 to 4, **characterized in that** said first containment body (2) is in the form of a lid which can be coupled onto said second containment body (3) for define said closing configuration (A); said first containment body (2) being provided with a first perimetral wall (22) which extends from a first closed bottom wall (21); said second containment body (3) being provided with a second perimetral wall (32) arranged internally to the first perimetral wall (22) of said first containment body (2) and which extends from a second bottom wall (31) provided with said first opening (32A).

7. Method for the sterilization of instruments for medical use using a cassette (1) according to any one of claims 1 to 9, **characterized by** the following operating phases:
- preparation of at least one said support tray (4) with one or more medical instruments to be sterilized supported on it;
- coupling of said first and second containment bodies (2, 3) to define said closed configuration (A) housing inside said housing chamber (100) said support tray (4) supporting the instruments for medical use to be sterilized; said coupling determining the formation of a cassette (1) equipped with instruments to be sterilized and providing the juxtaposition of said first peripheral edge (20) and second peripheral edge (30) with the interposition of said first sealing means (6) and the juxtaposition of said first and second frames (32, 40) with each other with the interposition of said at least one vapor-permeable sheet-like element (5), to seal in a sealed manner the flow passage (V) of said first opening (32A) and second opening (40A);
- insertion of said cassette (1) equipped with instruments to be sterilized inside an autoclave and start of an autoclave sterilization procedure in which at least one fluid in vapor state passes through the flow passage (V) and sterilizes the instruments at medical use to be sterilized supported by said support tray (4), obtaining a cassette (1) set up with sterilized medical instruments;
- extraction of said cassette (1) equipped with sterilized instruments from said autoclave.

8. Method for the sterilization of instruments for medical use according to claim 7, and by means of a cassette (1) according to claim 5, **characterized in that** the juxtaposition of said first and second frames (32, 40) with the interposition of said at least one vapor-permeable sheet-like element (5) involves first the arrangement of said sheet-like element (5) on said second frame (40) and then the subsequent overlapping of said second containment body (3) in the form of a lid on said first containment body (2) with the interposition of said sheet-like element (5) between said first and second frames (32, 40) opposite each other.

9. Method for the sterilization of instruments for medical use according to claim 7, and by means of a cassette according to claim 6, **characterized by** the fact that the juxtaposition of said first and second frames (32, 40) with the interposition of said at least one vapor-permeable sheet-like element (5) involves first the arrangement of said sheet-like element (5) on the second bottom wall (31) of said second containment body (3) in correspondence with said first opening (32A), and then the subsequent overlapping of said support tray (4) on said second bottom wall (31) with the interposition of said sheet-like element (5) between said first and second frames (32, 40) opposite each other.

10. Method for the sterilization of instruments for medical use according to claim 8, **characterized in that** said support tray (4) equipped with instruments for medical use to be sterilized is subjected to a mechanical washing phase in an equipment for cleaning with liquids at high temperature or ultrasound before said coupling phase of said first and second containment bodies (2, 3).
